(19) Europäisches Patentamt
European Patent Office
Office européen des brevets

(11) Publication number: **0 049 631 B1**

# (12) EUROPEAN PATENT SPECIFICATION

(45) Date of publication of patent specification: **22.02.84**

(21) Application number: **81304610.9**

(22) Date of filing: **05.10.81**

(51) Int. Cl.³: **C 07 C 31/27,** C 07 C 33/05, C 07 C 47/36, C 07 C 47/46, C 07 C 29/14, C 07 C 29/38, C 07 C 45/50, C 08 G 18/32

## (54) Polycyclic alcohols.

(30) Priority: **06.10.80 US 194172**

(43) Date of publication of application: **14.04.82 Bulletin 82/15**

(45) Publication of the grant of the patent: **22.02.84 Bulletin 84/8**

(84) Designated Contracting States: **AT BE CH DE FR GB IT LI LU NL SE**

(56) References cited:
DE - A - 2 013 316
DE - B - 1 618 384
US - A - 4 146 505
US - A - 4 216 343

Patent Abstracts of Japan Vol. 4, No. 7, 19 January 1980 page 150C70

(73) Proprietor: **HENKEL CORPORATION**
**4620 West 77th Street**
**Minneapolis Minnesota 55435 (US)**

(72) Inventor: **Rogier, Edgar Robert**
**16400 Hidden Valley Road**
**Minnetonka Minnesota 55434 (US)**

(74) Representative: **Watkins, Arnold Jack et al,**
**European Patent Attorney Frank B. Dehn & Co.**
**Imperial House 15-19 Kingsway**
**London WC2B 6UZ (GB)**

EP 0 049 631 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Polycyclic alcohols

The present invention relates to polycyclic compounds having a gem bis(hydroxymethyl) or hydroxymethyl formyl structure. The compounds of the invention may be utilized for curing into various thermoplastic or thermosetting compositions. The compounds may be saturated or ethylenically unsaturated or may be in the form of halogen or phosphite derivatives to obtain fire retardant properties.

Various tricyclic compounds are described in United States Patent 3,470,248 issued September 30, 1969 to Brotherton et al. The materials described in the Brotherton et al patent are stated to be useful in the resin art such as in the preparation of urethane polymers, polyamides and polyurethane polyurea elastomers. United States Patent 3,787,371 issued January 22, 1974 to Brinkmann et al discloses similar compounds which are stated to be useful in the formation of clear polyamides.

DE—B—1618384 (Ruhrchemie AG) describes a process in which dicyclopentadiene is hydroformylated over a rhodium catalyst and the aldehyde product is subsequently reduced. In the process, hydroformylation will occur at both of the carbon carbon double bonds in the dicyclopentadiene starting product resulting in the diol end product 2(3), 8(9) bis (hydroxymethyl) tricyclo $(5,2,1,0^{2,6})$ decane.

United States Patent 3,317,469 issued May 2, 1967 to Feichtinger et al also discloses the use of materials similar to those in the Brotherton patent. Wagner et al in German OLS 2641662 published March 23, 1978 also discloses substituted tricyclodecane derivatives. British Patent 1,266,016 published March 8, 1972 discloses tricyclodecane curing agent. Japanese published patent application 54—4992 published January 16, 1979 naming Kaya as an inventor also describes polycyclic compounds. Such compounds are also described in European patent application publication No. 26983A of Henkel Corporation. Additional work concerning such compounds is found in the disclosures of Fujikura et al Synthetic Communications Volume 6 No. 3 pages 199—207 (1976). Further disclosures of such technology concerning polycyclic compounds is found in Pruett, Ann. N.Y., Acad. Sci. Volume 295 pages 239—248 (1977).

Additional polycyclic compounds are disclosed in German OLS 2,200,022 published July 19, 1973 by Gierenz et al. Still further technology involving polycyclic compounds is found German OLS 2,307,627 published September 5, 1974 by Grau. German OLS 2,013,316 published October 1, 1979 by von Bornhaupt also discloses polycyclic derivatives having hydroxyl functionality.

German Patent 934,889 granted November 19, 1955 to Roelen et al discloses polyesters of certain polycyclic compounds. German Patent 1,694,868 granted February 19, 1972 to Jellienk et al discloses the use of polycyclic polyhydroxyl functional compounds in the preparation of urethanes.

Polycyclic compounds which are used to prepare unsaturated esters such as with maleic acid are disclosed in German OLS 1,916,287 dated October 15, 1970 to Kolbel et al.

Several additional polycyclic compounds are disclosed in German OLS 2,200,021, published July 26, 1973 naming Vegemund as an inventor, which discloses additional polycyclic polyfunctional compounds.

Canadian Patent 893,716 to Falbe issued February 22, 1972 also discloses aldehydes manufactured from polycyclic compounds. Conjugated unsaturated compounds, which are polycyclic in nature, are described in United States Patent 4,143,065 issued March 6, 1979 to Hoffmann et al. Additional compounds of interest to chemists studying polycyclic materials are described in United States Patent 4,146,505 issued March 27, 1979 to Weber et al. Certain aldehydes of polycyclic compounds are also disclosed in British Patent 734,030 published July 2, 1955. Canadian Patent 867,229 also describes the production of polycyclic polyols in an application published May 3, 1961.

Nyi in United States Patent 4,140,724 describes certain polycyclic monoethers in a patent granted February 20, 1979. In United States Patent 4,117,030 issued September 26, 1978 to Nelson several additional polycyclic compounds having various functional groups are disclosed.

Even though substantial work has been done in the area of polycyclic compounds, it has not previously been recognized that superior properties may be obtained from the *geminal* bis(hydroxymethyl) structure on the polycyclic compounds of the present invention and the fire retardant materials formed from derivatives of such compounds.

Although United States Patent 4216343, issued August 5 1980 to Rogier, describes certain gem-bis(hydroxymethyl)- group containing polyols as being useful in the production of polyurethanes, the particular polyols are straight chain aliphatic $C_{14-22}$ alkan-l-ols substituted at one, two or three positions along the chain by gem-bis-(hydroxymethyl) functionalities. These gem-bis-(hydroxymethyl) alkan-l-ols are described as being advantageous as polyols inter alia as a result of the absence of tertiary hydrogens which would provide weak points for chemical attack on the molecule and there is no suggestion that the gem-bis-(hydroxymethyl) substitution of a polycyclic skeleton rather than of the straight chain alkan-l-ol skeleton might also result in advantageous polyols.

Throughout this specification percentages and ratios are given by weight and temperatures are in degrees Celsius unless otherwise indicated.

According to one aspect of the present invention we provide polycyclic compounds of formula I

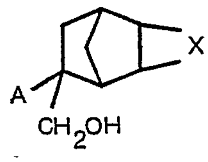

(I)

wherein A represents a $-CH_2OH$ or $-CHO$ group and X represents a group of formula $-CH{=}CH-CH_2-$, $-CH_2-CH_2-CH_2-$, $CHY-CH_2-CH_2-$, $-CH_2-CHY-CH_2-$ or

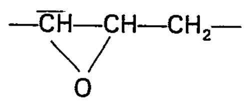

(in which Y represents a halogen atom or a phosphonate group).

Particularly preferred polycyclic alcohols of formula I are those wherein A represents a $-CH_2OH$ group and X represents a group of formula $-CH_2-CH_2-CH_2-$ or $-CH{=}CH-CH_2-$ or mixtures of these. These 8,8(9,9)-bis-(hydroxymethyl) tricyclo compounds have the formula IA or IB as shown below.

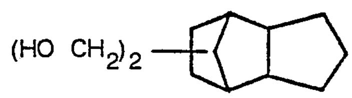

(IA)

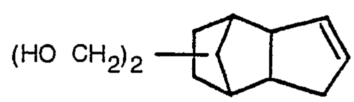

(IB)

The present invention is deemed to include within its scope individual compounds or stereoisomers of compounds of formula I as well as mixtures thereof.

The compounds of the present invention may be prepared from dicyclopentadiene (tricyclo [5,2,1,$0^{2,6}$] deca-3,8-diene). The structural formula for dicyclopentadiene showing the basic numbering system for dicyclopentadiene and dicyclopentadiene derivatives is as follows:

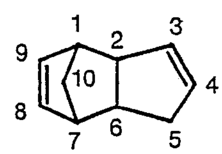

(II)

According to a further aspect of the present invention we therefore provide a process for the preparation of compounds of formula I which process comprises hydroformylating a compound of formula II,

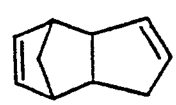

(II)

reacting the aldehyde obtained thereby with formaldehyde to produce a hydroxymethyl formyl compound of formula IV

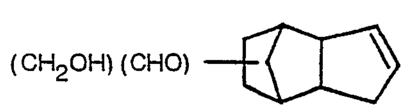

(IV)

and, if desired, performing one or both of the following steps (a) reducing the aldehyde radical and/or the ethylenically unsaturated moiety and (b) substituting at the 3- or 4- position with a halogen atom or an oxirane or phosphonate group to form a compound of formula I.

In the process of the invention, the parent dicyclopentadiene compound is reacted with hydrogen and carbon monoxide under selective conditions to place a formyl group on either the 8th or 9th carbon atoms and to place a hydrogen on the remaining carbon atom. The reaction conditions as hereinafter

described are quite selective so that the remaining bond between the 3rd and 4th carbon atom is not affected by this reaction. This is particularly important in that it may be desired that this remaining bond remain intact to allow for conversion for example to the (3 or 4) chloro or bromo and the 3(4) phosphonate derivatives. It is also noted that the unsaturation between the 3rd and 4th carbon atoms is, of course, useful as this facilitates formation of an oxirane group at the 3—4 position.

After the formyl group has been added in the 8(9) position, formaldehyde is employed to convert the formyl group to a hydroxymethyl formyl moeity. This, of course, does not change the positioning of the formyl group on the ring structure. The conditions for reactions with formaldehyde are also selected such that the unsaturation in the 3—4 position is not disturbed. After obtaining the hydroxymethyl formyl functionality in the 8(9) position, the formyl group may be reduced to give the *gem* bis-(hydroxymethyl) structure. The *gem* bis(hydroxymethyl) compound of formula IB thus obtained is properly named 8,8(9,9) bis(hydroxymethyl) tricyclo ($5,2,1,0^{2,6}$) dec-3-ene. The reduction to give the geminal compounds may be accomplished through using an additional mole of formaldehyde under alkaline conditions or the reduction may be done utilizing materials such as sodium borohydride.

The suggested conditions for adding the *gem* bis(hydroxymethyl) group to polycyclic dicyclopentadiene compounds involve hydroformylation of the 8—9 double bond to obtain the desired monoformyl compound. Conveniently, however, a slight stoichiometric excess may be added to ensure completeness of the reaction. The mixture of hydrogen and carbon monoxide for the hydroformylation reaction is conveniently maintained with respect to one another at from about 1.5:0.5 to about 0.5:1.5 on a molar ratio. It is noted that this ratio is not critical as long as the pressure is maintained in the reaction vessel by the component gases and that the amount of hydrogen is not so great as to substantially reduce any of the unsaturation in the ring system. The reaction sequence to obtain the products of formulae IA and IB is shown below.

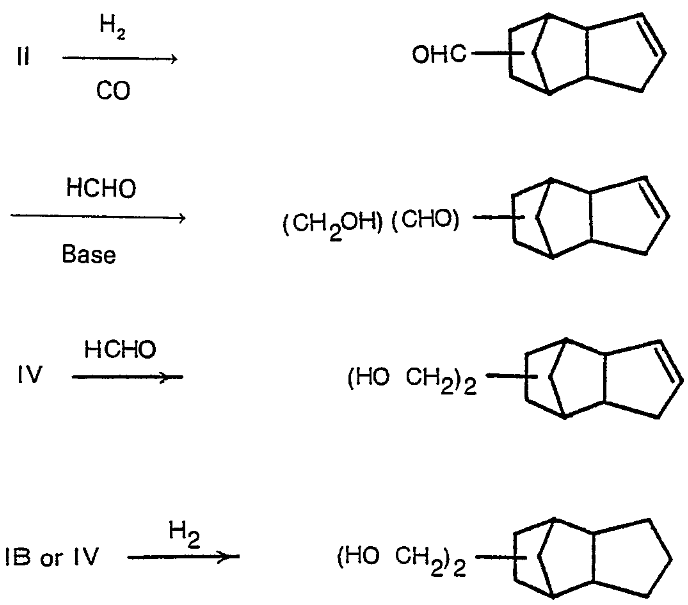


(III)

(IV)

(IB)

(IA)

The hydroformylation is accomplished most conveniently utilizing a catalyst such as rhodium. The source of the rhodium catalyst may be for example rhodium chloride, rhodium, dicarbonyl chloride dimer, rhodium carbonyl hydrides liganded by phosphines or phosphites, rhodium nitrate, rhodium trichloride and other materials. The phosphine or phosphite ligands are conveniently trisubstituted by alkyl or aryl groups, suitably aryl groups and preferably phenyl groups. Several additional ligands which may be suitable are discussed in "Selective Hydroformylation of Unsaturated Fatty Acid Esters" by Frankel in the Annals N.Y. Academy of Sciences 214:79 (1973). Suitable examples of such catalysts include many forms of rhodium, preferably liganded by for example trialkyl amine, triphenylphosphite or triphenylphosphine.

The hydroformylation stage of the process of the invention is conveniently conducted at from about 70 degrees C to 100 degrees C, preferably from about 80 degrees C to 90 degrees C. the pressure within the reaction vessel is conveniently maintained at from about 10 to 150 atmospheres, preferably from 80 to 100 atmospheres absolute.

The hydroformylated reaction product is then isolated, conveniently using distillation, leaving the residue containing the expensive catalyst. The formyl product (III) obtained from the hydroformylation is then reacted in the presence of base with formaldehyde to give the corresponding hydroxymethyl formyl compound (IV). The reaction may proceed utilzing weak base; alternatively, it is convenient to

utilize two moles of formaldehyde and strong base such as sodium hydroxide to push the reaction all the way to the *gem*-bis(hydroxymethyl) product (IB).

The reaction to obtain the hydroxymethyl formyl compound is best conducted in an inert atmosphere, particularly nitrogen. The *gem*-bis(hydroxymethyl) polycyclic compound so formed is washed with water to remove any excess caustic and salts formed and then obtained in a relatively pure state by distillation.

An alternative method of accomplishing the formation of the *gem*-bis(hydroxymethyl) polycyclic compounds is by utilizing only one-half the equivalent amount of formaldehyde required to obtain the *gem*-bis(hydroxymethyl) product directly. That is, the formyl compound is converted to the corresponding hydroxymethyl formyl polycyclic compound and is then reduced using a strong reductant, e.g. sodium borohydride or lithium aluminum hydride.

The *gem*-bis(hydroxymethyl) compounds of the present invention may be used to prepare urethane coatings and castings. For example, a urethane coating system may be formed by reacting the hydroxyls of compounds of formula IA or IB with a polyfunctional isocyanate to obtain the urethane linkage.

The unsaturated gem-bis(hydroxymethyl) compounds of the present invention may be hydrogenated to give the saturated gem-bis(hydroxymethyl) compounds of formula IA. Such saturated gem-bis(hydroxymethyl) compounds are useful where it is not desired to have unsaturation in the backbone of the particular composition, e.g. polyurethane or polyester. The hydroxymethyl formyl compounds of the present invention have the potential of being present as both the endo and exo isomers and are useful where both hydroxyl and aldehyde functionality are desired.

According to a still further feature of the present invention we provide a process for the preparation of polyurethanes which process comprises reacting a compound of formula I

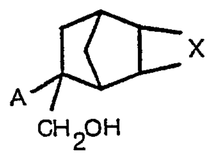

(I)

(wherein A represents a $-CH_2OH$ group and X is as hereinbefore defined) with a polyfunctional isocyanate.

The following non-limiting Examples are provided to illustrate the preparation of the compounds of the invention.

Example I

Preparation of 8(9) formyltricyclo [5,2,1,$0^{2,6}$] dec-3-ene

Into a 1 litre 316 SS autoclave was placed 105 g of dicyclopentadiene, 351 g of toluene, 1.0 g of 5% rhodium on alumina (Englehardt Industries) and 0.34 g of triphenylphosphite. The autoclave is purged with nitrogen then charged with a 1:1 mixture of carbon monoxide-hydrogen to a pressure of 65.85 bars (65 atmospheres). The autoclave is heated, with stirring, to about 70—80 degrees C where gas uptake begins. The temperature was maintained between 80—90 degrees C at 70.9 bars (70 atmospheres) for 1.7 hours. The autoclave is cooled to 50 degrees C and the product discharged through a filter. Distillation of the solvent produced 122 g of a compound of formula III having a carbonyl equivalent weight of 170. Infra-red and NMR spectra confirmed formula III to be the correct structure: G.C. analysis of this product showed this to contain 96% of the compound of formula III and 2.3% of diformyltricyclodecane derivatives.

Example II

Preparation of 8,8(9,9)-bis(hydroxymethyl)-tricyclo [5,2,1,$0^{2,6}$] dec-3-ene

Into a 250 ml, 3-necked flask is placed 77.6 g of an aldehyde of formula III (e.g. that produced according to Example I), and 57.8 g of a 55 percent solution of formaldehyde in methanol. The solution is cooled to 11 degrees C and 1 ml of 40 percent aqueous sodium hydroxide solution is added with stirring. The reaction mixture is gradually warmed to 45 degrees C over a period of one hour. Stirring is continued and 40 ml of 40 percent sodium hydroxide solution is added over a period of 47 minutes during which time the temperature is maintained at 45—62 degrees C. The reaction mixture is stirred for about three hours more and the temperature allowed to fall to 36 degrees C. Near the end of this time, 13 ml of a 12 percent solution of sodium borohydride in aqueous sodium hydroxide is added to convert any remainder of the hydroxymethyl formyl compound of formula IV to the bis-(hydroxymethyl) product of formula IB. Methanol and water are then removed under vacuum from the reaction mixture at 42 degrees C. The residue material is dissolved in a heated mixture of 200 ml toluene and 200 ml water. The hot toluene layer is separated and washed with water until neutral. Distillation of the toluene under reduced pressure yields 87.3 g of the bis(hydroxymethyl) compound of formula IB containing about 15 percent of 8(9)-hydroxymethyl tricyclo [5,2,1,$0^{2,6}$] dec-3-ene.

Fractional distillation of a similar crude sample of the title product yielded the title product at 99 percent purity (GC), b.p. 165 degrees C (66.66 Pa, 0.5 torr), m.p. 87 degrees C.

Example III

Preparation of 8,8(9,9)-bis(hydroxymethyl)-tricyclo $[5,2,1,0^{2,6}]$ decane

The compound of formula IB (e.g. that produced according to Example II) is hydrogenated to yield the saturated bis(hydroxymethyl) compound of formula IA by adding into a Parr reaction apparatus 19.4 grams of a compound of formula IB in 100 ml of absolute ethanol. One gram 5 percent palladium on carbon is then added and the system is sealed and flushed with nitrogen. Hydrogen gas is added at 4 atmospheres pressure and the flask is shaken until gas uptake is complete (about 1 hour). The catalyst is removed by filtration and the title product is recovered by evaporation of the ethanol. 19.3 grams of the title product of formula IA is recovered and has a melt point of 105 degrees C.

Example IV

The compound of formula IB is formed into a polyurethane film by reacting with L2291A, an aliphatic trifunctional isocyanate from Mobay, in a 1:1 equivalent ratio.

The film is cured at 66 degrees C for four hours on a Bondenite 1000 substrate at a 2 mm thickness. The pencil hardness is 2H—3H.

**Claims for the contracting states: BE, CH, DE, FR, GB, IT, LI, LU, NL, SE**

1. Polycyclic compounds of formula I

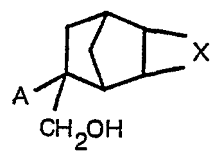

(I)

wherein A represents a $—CH_2OH$ or —CHO group and X represents a group of formula $—CH{=}CH—CH_2—$, $—CH_2—CH_2—CH_2—$, $—CHY—CH_2—CH_2—CH_2—$, $—CH_2—CHY—CH_2$ or

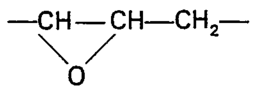

(in which Y represents a halogen atom or a phosphonate group).

2. Compounds of formula I as claimed in claim 1 wherein X represents a group of formula $—CH{=}CH—CH_2—$ or $—CH_2—CH_2—CH_2—$ and A represents a $—CH_2OH$ group.

3. Compounds of formula I as claimed in claim 1 wherein X represents a group of formula $—CH{=}CH—CH_2—$ and A represents a —CHO group.

4. A process for the preparation of compounds of formula I (as defined in claim 1) which process comprises hydroformylating a compound of formula II,

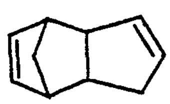

(II)

reacting the aldehyde obtained thereby with formaldehyde to produce a hydroxymethyl formyl compound of formula IV

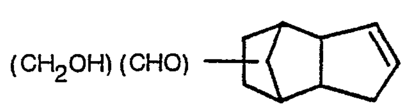

(IV)

and, if desired, performing one or both of the following steps (a) reducing the aldehyde radical and/or the ethylenically unsaturated moiety and (b) substituting at the 3- or 4-position with a halogen atom or an oxirane or phosphonate group to form a compound of formula I.

5. A process as claimed in claim 4 wherein the hydroformylation is effected in the presence of a rhodium catalyst.

6. A process as claimed in either of claims 4 and 5 wherein the hydroxymethyl formyl compound of formula IV is reduced with formaldehyde to a gem-bis(hydroxymethyl) compound of formula IB.

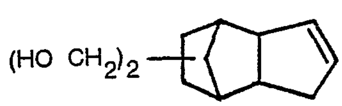

(IB)

7. A process as claimed in either of claims 4 and 5 wherein the hydroxymethyl formyl compound of formula IV is reduced with a strong reductant to a gem-bis(hydroxymethyl) compound of formula IA.

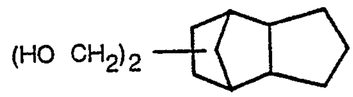
(IA)

8. A process for the preparation of polyurethanes which process comprises reacting a compound of formula I (wherein A represents a $—CH_2OH$ group and X is as defined in claim 1) with a polyfunctional isocyanate.

**Claims: for the contracting states: AT**

1. A process for the preparation of compounds of formula I

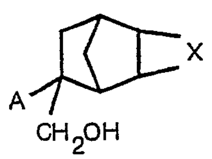
(I)

(wherein A represents a $—CH_2OH$ or —CHO group and X represents a group of formula $—CH{=}CH—CH_2—$, $—CH_2—CH_2—CH_2—$, $—CHY—CH_2—CH_2—$, $—CH_2—CHY—CH_2—$ or

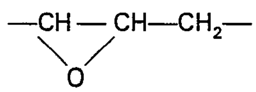

[in which Y represents a halogen atom or a phosphonate group]) which process comprises hydroformylating a compound of formula II,

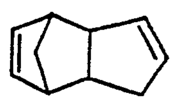
(II)

reacting the aldehyde obtained thereby with formaldehyde to produce a hydroxymethyl formyl compound of formula IV

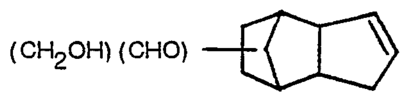
(IV)

and, if desired, performing one or both of the following steps (a) reducing the aldehyde radical and/or the ethylenically unsaturated moiety and (b) substituting at the 3- or 4- position with a halogen atom or an oxirane or phosphonate group to form a compound of formula I.

2. A process as claimed in claim 1 wherein the hydroformylation is effected in the presence of a rhodium catalyst.

3. A process as claimed in either of claims 1 and 2 wherein the hydroxymethyl formyl compound of formula IV is reduced with formaldehyde to a gem-bis-hydroxymethyl compound of formula IB.

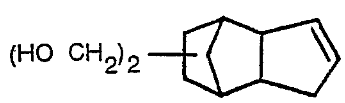
(IB)

4. A process as claimed in either of claims 1 and 2 wherein the hydroxymethyl formyl compound of formula IV is reduced with a strong reductant to a gem-bis-hydroxymethyl compound of formula IA.

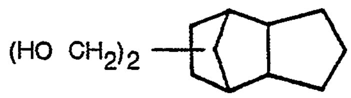

(IA)

5. A process for the preparation of polyurethanes which process comprises reacting a compound of formula I (wherein A represents a —$CH_2OH$ group and X is as defined in claim 1) with a polyfunctional isocyanate.

**Revendications pour les Etats contractants: BE CH LI DE FR GB IT LU NL SE**

1. Composés polycycliques de formule I

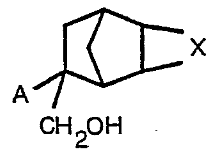

(I)

dans laquelle A représente un groupe —$CH_2OH$ ou —CHO et X représente un groupe de formule —CH=CH—$CH_2$—, —$CH_2$—$CH_2$—$CH_2$—, —CHY—$CH_2$—$CH_2$—, —$CH_2$—CHY—$CH_2$— ou

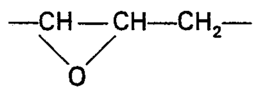

(où Y représente un atome d'halogène ou un groupe phosphonate).

2. Composés de formule I selon la revendication 1, dans lesquels X représente un groupe de formule —CH=CH—$CH_2$— ou —$CH_2$—$CH_2$—$CH_2$— et A représente un groupe —$CH_2OH$.

3. Composés de formule I selon la revendication 1 dans lesquels X représente un groupe de formule —CH=CH—$CH_2$— et A représente un groupe —CHO.

4. Procédé pour la préparation de composés de formule I (comme définis à la revendication 1), procédé qui consiste à hydroformyler un composé de formule II,

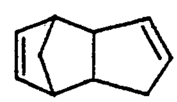

(II)

à faire réagir l'aldéhyde ainsi obtenu avec le formaldéhyde pour produire un composé hydroxyméthylé formylé de formule IV

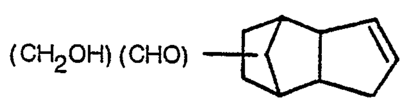

(IV)

et, si on le désire à effectuer l'une ou les deux étapes suivantes: (a) réduction du radical aldéhyde et/ou du reste à insaturation éthylénique et (b) substitution en position 3- ou 4- par un halogène ou un groupe oxiranne ou phosphonate pour obenir un composé de formule I.

5. Procédé selon la revendication 4, dans lequel l'hydroformylation est effectuée en présence d'un catalyseur au rhodium.

6. Procédé selon l'une des revendications 4 et 5, dans lequel le composé hydroxyméthylé formylé de formule IV est réduit par le formaldéhyde en un composé gem-bis(hydroxyméthyl) de formule IB

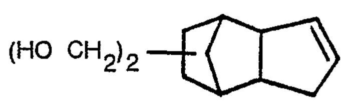

(IB)

7. Procédé selon l'une des revendications 4 et 5, dans lequel le composé hydroxyméthylé formylé de formule IV est réduit par un réducteur fort en un composant gem-bis(hydroxyméthyl) de formule IA

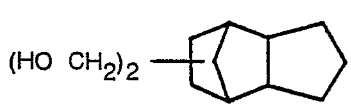

(IA)

8. Procédé pour la préparation de polyuréthannes, qui consiste à faire réagir un composé de formule I (où A représente un groupe $—CH_2OH$ et X est comme défini à la revendication 1) avec un isocyanate polyfonctionnel.

**Revendications pour l'etat contractant: AT**

1. Procédé pour la préparation de composés formule I

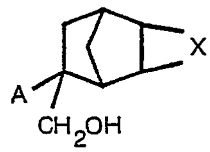

(I)

dans laquelle A représente un groupe $—CH_2OH$ ou —CHO et X représente un groupe de formule $—CH{=}CH—CH_2—$, $—CH_2—CH_2—CH_2—$, $—CHY—CH_2—CH_2—$, $—CH_2—CHY—CH_2—$ ou

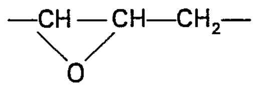

(où Y représente un atome d'halogène ou un groupe phosphonate), procédé qui consiste à hydroformyler un composé de formule II

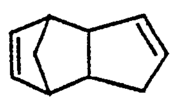

(II)

à faire réagir l'aldéhyde ainsi obtenu avec le formaldéhyde pour produire un composé hydroxyméthylé formylé de formule IV

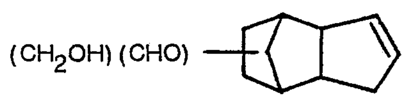

(IV)

et, si on le désire, à effectuer l'une ou les deux étapes suivantes: (a) réduction du radical aldéhyde et/ou du reste à insaturation éthylénique et (b) substitution en position 3- ou 4- par un halgène ou un groupe oxiranne ou phosphonate pour obtenir un composé de formule I.

2. Procédé selon la revendication 1, dans lequel l'hydroformylation est effectuée en présence d'un catalyseur au rhodium.

3. Procédé selon l'une des revendications 1 et 2, dans lequel le composé hydroxyméthylé formylé de formule IV est réduit par le formaldéhyde en un composé gem-bis(hydroxyméthyl) de formule IB

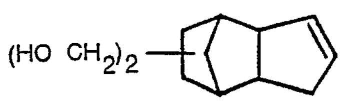

(IB)

4. Procédé selon l'une des revendications 1 et 2, dans lequel le composé hydroxyméthylé formylé de formule IV est réduit par un réducteur fort en un composant gem-bis(hydroxyméthyl) de formule IA

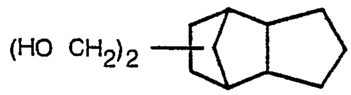

(IA)

5. Procédé pour la préparation de polyuréthannes, qui consiste à faire réagir un composé de formule I (où A représente un groupe $—CH_2OH$ et X est comme défini à la revendication 1) avec un isocyanate polyfonctionnel.

**Patentansprüche für die vertragstaaten BE, CH, DE, FR, GB, IT, LI, LU, NL, SE**

1. Polycyclische Verbindungen der Formel I

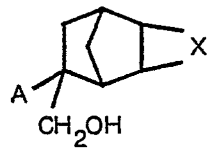

(I)

worin A eine Gruppe $—CH_2OH$ oder —CHO darstellt und X eine Gruppe der Formel $—CH{=}CH—CH_2—$, $—CH_2—CH_2—CH_2—$, $—CHY—CH_2—CH_2—$, $—CH_2—CHY—CH_2—$ oder

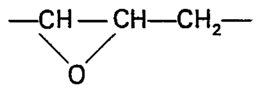

(wobei Y ein Halogenatom oder eine Phosphonatgruppe ist) bedeutet.

2. Verbindungen der Formel I, wie in Anspruch 1 beansprucht, worin X eine Gruppe der Formel $—CH{=}CH—CH_2—$ oder $—CH_2—CH_2—CH_2—$ darstellt und A eine Gruppe $—CH_2OH$ bedeutet.

3. Verbindungen der Formel I, wie in Anspruch 1 beansprucht, worin X eine Gruppe der Formel $—CH{=}CH—CH_2—$ darstellt und A eine Gruppe —CHO bedeutet.

4. Ein Verfahren zur Herstellung von Verbindungen der Formel I (wie in Anspruch 1 definiert), welches Verfahren die Hydroformylierung einer Verbindung der Formel II

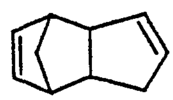

(II)

die Umsetzung des dadurch erhaltenen Aldehyds mit Formaldehyd unter Bildung einer Hydroxymethylformylverbindung der Formel

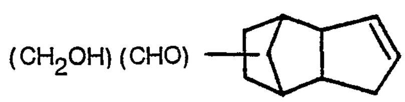

(IV)

und, wenn gewünscht, die Durchführung eines oder beider der folgenden Schritte (a) Reduzieren des Aldehydrestes und/oder der äthylenisch ungesättigten Gruppe und (b) Substitution in Stellung 3 oder 4 mit einem Halogenatom oder einer Oxiran- oder Phosphonat-gruppe umfaßt zur Bildung einer Verbindung der Formel I.

5. Verfahren wie in Anspruch 4 beansprucht, wobei die Hydroformylierung in Anwesenheit eines Rhodiumkatalysators bewirkt wird.

6. Verfahren wie in einem der Ansprüche 4 und 5 beansprucht, wobei die Hydroxymethylformylverbindung der Formel (IV) mit Formaldehyd zu einer gem-bis(Hydroxymethyl)-verbindung der Formel IB

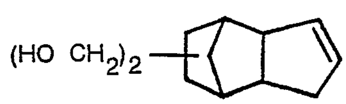

(IB)

reduziert wird.

7. Verfahren wie in einem der Ansprüche 4 und 5 beansprucht, wobei die Hydroxymethylformylverbindung der Formel (IV) mit einem starken Reduktionsmittel zu einer gem-bis(Hydroxymethyl)-verbindung der Formel IA

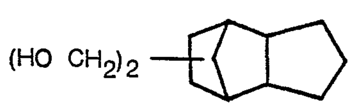

(IA)

reduziert wird.

8. Verfahren zur Herstellung von Polyurethanen, welches Verfahren die Umsetzung einer Verbindung der Formel I (worin A eine Gruppe $—CH_2OH$ darstellt und X wie in Anspruch 1 definiert ist) mit einem polyfunktionellen Isocyanat umfaßt.

**Patentansprüche für den Vertragsstaat: AT**

1. Verfahren zur Herstellung von Verbindungen der Formel I

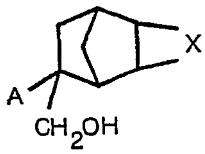

(I)

worin A eine Gruppe $—CH_2OH$ oder —CHO darstellt und X eine Gruppe der Formel $—CH{=}CH—CH_2—$, $—CH_2—CH_2—CH_2—$, $—CHY—CH_2—CH_2—$, $—CH_2—CHY—CH_2—$ oder

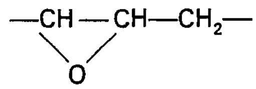

(wobei Y ein Halogenatom oder eine Phosphonatgruppe ist) bedeutet, welches Verfahren die Hydroformylierung einer Verbindung der Formel II

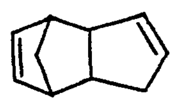

(II)

die Umsetzung des dadurch erhaltenen Aldehyds mit Formaldehyd unter Bildung einer Hydroxymethylformylverbindung der Formel IV

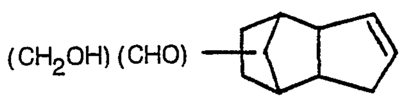

(IV)

und, wenn gewünscht, die Durchführung eines oder beider der folgenden Schritte (a) Reduzieren des Aldehydrestes und/oder der äthylensich ungesättigten Gruppe und (b) Substitution in Stellung 3 oder 4 mit einem Halogenatom oder einer Oxiran- oder Phosphonat-gruppe umfaßt zur Bildung einer Verbindung der Formel I.

2. Verfahren wie in Anspruch 1 beansprucht, wobei die Hydroformylierung in Anwesenheit eines Rhodiumkatalysators bewirkt wird.

3. Verfahren wie in einem der Ansprüche 1 und 2 beansprucht, wobei die Hydroxymethylformylverbindung der Formel (IV) mit Formaldehyd zu einer gem-bis(Hydroxymethyl)-verbindung der Formel IB

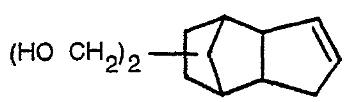

(IB)

reduziert wird.

4. Verfahren wie in einem der Ansprüche 1 und 2 beansprucht, wobei die Hydroxymethylformylverbindung der Formel (IV) mit einem starken Reduktionsmittel zu einer gem-bis(Hydroxymethyl)-verbindung der Formel IA

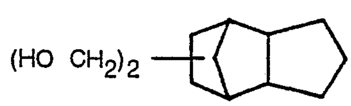

(IA)

reduziert wird.

5. Verfahren zur Herstellung von Polyurethanen, welches Verfahren die Umsetzung einer Verbindung der Formel I (worin A eine Gruppe $—CH_2OH$ darstellt und X wie in Anspruch 1 definiert ist) mit einem polyfunktionellen Isocyanat umfaßt.